# EUROPEAN PATENT APPLICATION

(11) **EP 4 830 733 A1**
(43) Date of publication of application: **28.10.2026**
(21) Application number: 24906511.1
(22) Date of filing: 20.12.2024
(51) Int. Cl.: A61K 47/68, C07K 16/32, C07K 16/46, A61P 35/00, A61K 47/54

(54) **USE OF ADC TARGETING HER2 IN TREATMENT OF BREAST CANCER**

(30) Priority: 22.12.2023 CN 202311781043
(71) Applicant: Chia Tai Tianqing Pharmaceutical Group Co., Ltd., Lianyungang, Jiangsu 222062 (CN)
(72) Inventor: HU, Yanyan, Lianyungang, Jiangsu 222062 (CN); ZHANG, Xiquan, Lianyungang, Jiangsu 222062 (CN); WANG, Xunqiang, Lianyungang, Jiangsu 222062 (CN); LI, Xu, Lianyungang, Jiangsu 222062 (CN); LI, Zhenling, Lianyungang, Jiangsu 222062 (CN)
(74) Representative: v. Bezold & Partner Patentanwälte - PartG mbB
(86) International application number: PCT/CN2024/140881
(87) International publication number: WO 2025/131036

(57) **Abstract**

The present disclosure relates to the field of biomedicine, and relates to a use of an ADC targeting HER2 in the treatment of breast cancer. The present disclosure also relates to a method for treating breast cancer in a subject, said method comprising: administering an anti-HER2 ADC to a subject. The present disclosure further relates to a use of the anti-HER2 ADC in the preparation of a drug for neoadjuvant therapy of breast cancer. The method is beneficial to breast cancer subjects and has good safety.

## Description

### TECHNICAL FIELD

The present disclosure pertains to the field of biomedicine and particularly relates to use of an HER2-targeting ADC (antibody-drug conjugate) for treating breast cancer.

### BACKGROUND

Human epidermal growth factor receptor 2 (HER2) belongs to the human epidermal growth factor receptor family, which includes EGFR (ErbB-1), HER2/c-neu (ErbB-2), HER3 (ErbB-3), and HER4 (ErbB-4). These receptors are all located on the cell surface and have similar structures. As a widely expressed receptor protein, HER2 exhibits abnormal gene amplification that leads to protein overexpression, which in turn causes abnormal activation of signaling pathways, serving as one of the main pathways for promoting the growth of solid tumors. HER2 can form homodimers and is also prone to forming heterodimers with other receptor members of the HER family, leading to phosphorylation of receptor tyrosine residues and initiation of various signaling pathways, including MPK, PI3K, JAK, STAT3, PKC, and the like, thereby resulting in cell proliferation and tumorigenesis.

Anti-HER2 targeted therapy is an important approach for treating breast cancer; therefore, there is an urgent need to explore drugs targeting HER2 to meet the huge clinical demand for breast cancer treatment.

### SUMMARY

### Methods for Treating Breast Cancer

The present disclosure provides a method for treating breast cancer in a subject, which comprises administering to the subject the anti-HER2 antibody-drug conjugate of the present disclosure. In some embodiments, the method comprises administering to the subject the anti-HER2 antibody-drug conjugate of the present disclosure prior to surgical treatment. In some embodiments, the method comprises: (1) administering to the subject the anti-HER2 antibody-drug conjugate of the present disclosure; and (2) treating the breast cancer by surgical treatment.

The present disclosure further provides a method for neoadjuvant therapy of breast cancer in a subject, which comprises administering to the subject the anti-HER2 antibody-drug conjugate of the present disclosure. In some embodiments, the method is used in combination with breast cancer resection for the treatment of breast cancer in the subject. In some embodiments, after implementation of the method, breast cancer resection is further performed on the subject for the treatment of breast cancer.

The present disclosure further provides a method for treating early-stage or locally advanced breast cancer in a subject, which comprises administering to the subject the anti-HER2 antibody-drug conjugate of the present disclosure.

In some embodiments, in the method described above, the anti-HER2 antibody-drug conjugate is administered in a therapeutically effective amount.

The present disclosure further provides use of the anti-HER2 antibody-drug conjugate of the present disclosure for preparing a medicament for neoadjuvant therapy of breast cancer. In some embodiments, the medicament comprises a therapeutically effective amount of the anti-HER2 antibody-drug conjugate.

The present disclosure further provides an anti-HER2 antibody-drug conjugate for use in neoadjuvant therapy of breast cancer.

The present disclosure further provides the anti-HER2 antibody-drug conjugate of the present disclosure for use in treating breast cancer in a subject. The present disclosure further provides the anti-HER2 antibody-drug conjugate of the present disclosure for use in treating early-stage or locally advanced breast cancer in a subject. In some embodiments, the anti-HER2 antibody-drug conjugate is administered to the subject in a therapeutically effective amount.

The present disclosure further provides use of the anti-HER2 antibody-drug conjugate of the present disclosure for neoadjuvant therapy of breast cancer.

In some embodiments, in the method or the use, the anti-HER2 antibody-drug conjugate is administered once every 1 week (q1w), every 2 weeks (q2w), every 3 weeks (q3w), or every 4 weeks (q4w). In one specific embodiment, in the method or the use, the anti-HER2 antibody-drug conjugate is administered once every 3 weeks. In some embodiments, in the method or the use, the anti-HER2 antibody-drug conjugate is administered at a dose of 1.5-9 mg/kg, 4.5-7.5 mg/kg, or 6-7.5 mg/kg each time. In some embodiments, in the method or the use, the anti-HER2 antibody-drug conjugate is administered at a dose of 1.5 mg/kg, 3 mg/kg, 4.5 mg/kg, 6 mg/kg, 7.5 mg/kg, 9 mg/kg, or a range formed by any of the above values each time. In some embodiments, in the method or the use, the anti-HER2 antibody-drug conjugate is administered at a dose of 6 mg/kg or 7.5 mg/kg each time. In some embodiments, in the method or the use, the anti-HER2 antibody-drug conjugate is administered at a dose of 6 mg/kg each time. In some embodiments, in the method or the use, the anti-HER2 antibody-drug conjugate is administered at a dose of 7.5 mg/kg each time. In some embodiments, in the method or the use, the anti-HER2 antibody-drug conjugate is administered once every 1 week, every 2 weeks, every 3 weeks, or every 4 weeks at a dose of 1.5-9 mg/kg, 4.5-7.5 mg/kg, or 6-7.5 mg/kg of the anti-HER2 antibody-drug conjugate each time. In some embodiments, in the method or the use, the anti-HER2 antibody-drug conjugate is administered once every 3 weeks at a dose of 1.5 mg/kg, 3 mg/kg, 4.5 mg/kg, 6 mg/kg, 7.5 mg/kg, or 9 mg/kg of the anti-HER2 antibody-drug conjugate each time. In some embodiments, in the method or the use, the anti-HER2 antibody-drug conjugate is administered once every 3 weeks at a dose of 6 mg/kg or 7.5 mg/kg of the anti-HER2 antibody-drug conjugate each time. In some embodiments, in the method or the use, the anti-HER2 antibody-drug conjugate is administered once every 3 weeks at a dose of 6 mg/kg of the anti-HER2 antibody-drug conjugate each time. In some embodiments, in the method or the use, the anti-HER2 antibody-drug conjugate is administered once every 3 weeks at a dose of 7.5 mg/kg of the anti-HER2 antibody-drug conjugate each time.

In some embodiments, in the method or the use, one treatment cycle consists of 1 week, 2 weeks, 3 weeks, or 4 weeks. In some embodiments, in the method or the use, one treatment cycle consists of 3 weeks. In some embodiments, in the method or the use, one treatment cycle consists of 1 week, 2 weeks, 3 weeks, or 4 weeks, and the anti-HER2 antibody-drug conjugate is administered once per treatment cycle. In some embodiments, in the method or the use, one treatment cycle consists of 3 weeks, and the anti-HER2 antibody-drug conjugate is administered once per treatment cycle. In some embodiments, in the method or the use, the anti-HER2 antibody-drug conjugate is administered for a total of 6-8 treatment cycles. In some embodiments, in the method or the use, the anti-HER2 antibody-drug conjugate is administered for a total of 6 treatment cycles or 8 treatment cycles. In some embodiments, in the method or the use, the anti-HER2 antibody-drug conjugate is administered for a total of 6 treatment cycles. In some embodiments, in the method or the use, the anti-HER2 antibody-drug conjugate is administered for a total of 8 treatment cycles.

In some embodiments, one treatment cycle consists of 3 weeks, and the anti-HER2 antibody-drug conjugate is administered continuously or intermittently for 6-8 treatment cycles. In some embodiments, one treatment cycle consists of 3 weeks, and the anti-HER2 antibody-drug conjugate is administered continuously for 6-8 treatment cycles. In some embodiments, one treatment cycle consists of 3 weeks, and the anti-HER2 antibody-drug conjugate is administered intermittently in a regular pattern for 6-8 treatment cycles (e.g., treatment is suspended for one treatment cycle after 2-3 treatment cycles of administration). In some embodiments, one treatment cycle consists of 3 weeks, and the anti-HER2 antibody-drug conjugate is administered intermittently in an irregular pattern for 6-8 treatment cycles.

In some embodiments, in the method or the use, one treatment cycle consists of 3 weeks, and the anti-HER2 antibody-drug conjugate is administered in each treatment cycle for a total of 6 treatment cycles or 8 treatment cycles. In some embodiments, in the method or the use, one treatment cycle consists of 3 weeks, and the anti-HER2 antibody-drug conjugate is administered on day 1 of each treatment cycle for a total of 6 treatment cycles or 8 treatment cycles. In some embodiments, in the method or the use, one treatment cycle consists of 3 weeks, and the anti-HER2 antibody-drug conjugate is administered at 6 mg/kg or 7.5 mg/kg in each treatment cycle for a total of 6 treatment cycles or 8 treatment cycles. In some embodiments, in the method or the use, one treatment cycle consists of 3 weeks, and the anti-HER2 antibody-drug conjugate is administered at 6 mg/kg or 7.5 mg/kg on day 1 of each treatment cycle for a total of 6 treatment cycles or 8 treatment cycles. In some embodiments, in the method or the use, one treatment cycle consists of 3 weeks, and the anti-HER2 antibody-drug conjugate is administered at 6 mg/kg in each treatment cycle for a total of 6 treatment cycles. In some embodiments, in the method or the use, one treatment cycle consists of 3 weeks, and the anti-HER2 antibody-drug conjugate is administered at 6 mg/kg on day 1 of each treatment cycle for a total of 6 treatment cycles. In some embodiments, in the method or the use, one treatment cycle consists of 3 weeks, and the anti-HER2 antibody-drug conjugate is administered at 6 mg/kg in each treatment cycle for a total of 8 treatment cycles. In some embodiments, in the method or the use, one treatment cycle consists of 3 weeks, and the anti-HER2 antibody-drug conjugate is administered at 6 mg/kg on day 1 of each treatment cycle for a total of 8 treatment cycles. In some embodiments, in the method or the use, one treatment cycle consists of 3 weeks, and the anti-HER2 antibody-drug conjugate is administered at 7.5 mg/kg in each treatment cycle for a total of 6 treatment cycles. In some embodiments, in the method or the use, one treatment cycle consists of 3 weeks, and the anti-HER2 antibody-drug conjugate is administered at 7.5 mg/kg on day 1 of each treatment cycle for a total of 6 treatment cycles. In some embodiments, in the method or the use, one treatment cycle consists of 3 weeks, and the anti-HER2 antibody-drug conjugate is administered at 7.5 mg/kg in each treatment cycle for a total of 8 treatment cycles. In some embodiments, in the method or the use, one treatment cycle consists of 3 weeks, and the anti-HER2 antibody-drug conjugate is administered at 7.5 mg/kg on day 1 of each treatment cycle for a total of 8 treatment cycles.

The anti-HER2 antibody-drug conjugate may be formulated together with one or more pharmaceutically acceptable excipients to prepare a suitable pharmaceutical composition (or referred to as a formulation). The pharmaceutical composition may be in any suitable dosage form. In some embodiments, the anti-HER2 antibody-drug conjugate is formulated into a formulation for parenteral administration. In some specific embodiments, the anti-HER2 antibody-drug conjugate is formulated into a formulation for intravenous, intramuscular, or other parenteral administration. In some specific embodiments, the anti-HER2 antibody-drug conjugate may be formulated into an injection. In some specific embodiments, the anti-HER2 antibody-drug conjugate is formulated into a formulation for intravenous injection or infusion.

In some embodiments, in the method or the use, the anti-HER2 antibody-drug conjugate is administered by intravenous infusion.

In the method or the use, the administration regimen (e.g., administration cycle, administration time, administration dose, and dose adjustment) of the anti-HER2 antibody-drug conjugate may be adjusted according to the severity of the disease, the response to the disease, any treatment-related toxicity, and the age and health status of a patient. For example, the anti-HER2 antibody-drug conjugate may be administered with a delay of 1-56 days, e.g., a delay of 1 week, 2 weeks, 3 weeks, 4 weeks, 5 weeks, 6 weeks, 7 weeks, or 8 weeks. For another example, the administration dose of the anti-HER2 antibody-drug conjugate may be adjusted from 6 mg/kg to 4.5 mg/kg, or from 7.5 mg/kg to 6 mg/kg.

In some embodiments, the breast cancer is early-stage breast cancer. In some embodiments, the breast cancer is locally advanced breast cancer.

In some embodiments, the breast cancer is invasive breast cancer. In some embodiments, the invasive breast cancer does not include bilateral invasive breast cancer. In some embodiments, the breast cancer is unilateral invasive breast cancer.

In some embodiments, the breast cancer is HER2-expressing breast cancer. In some embodiments, the breast cancer is HER2-positive breast cancer. In some embodiments, the HER2-positive breast cancer is breast cancer determined to have HER2 expression of 3+ by immunohistochemistry (IHC) (i.e., IHC 3+). In some embodiments, the HER2-positive breast cancer is breast cancer determined to have HER2 expression of 2+ by IHC and determined to be positive for HER2 expression by in situ hybridization (ISH) (i.e., IHC 2+ and ISH+). In some embodiments, the HER2-positive breast cancer is breast cancer determined to be positive for HER2 expression by ISH (e.g., by FISH dual-probe detection) (i.e., ISH+).

In some embodiments, the breast cancer is HER2-expressing early-stage or locally advanced breast cancer. In some embodiments, the breast cancer is HER2-positive early-stage or locally advanced breast cancer. In some embodiments, the breast cancer is HER2-expressing early-stage breast cancer. In some embodiments, the breast cancer is HER2-positive early-stage breast cancer. In some embodiments, the breast cancer is HER2-expressing locally advanced breast cancer. In some embodiments, the breast cancer is HER2-positive locally advanced breast cancer.

In some embodiments, the subject with breast cancer is positive for the hormone receptor (HR). In some embodiments, the subject with breast cancer is positive for the estrogen receptor (ER). In some embodiments, the subject with breast cancer is positive for the progesterone receptor (PR). In some embodiments, the subject with breast cancer is negative for HR. In some embodiments, the breast cancer is HER2-positive and hormone receptor-positive breast cancer. In some embodiments, the breast cancer is HER2-positive and hormone receptor-positive early-stage breast cancer. In some embodiments, the breast cancer is HER2-positive and hormone receptor-positive locally advanced breast cancer. In some embodiments, the breast cancer is HER2-positive and hormone receptor-negative breast cancer. In some embodiments, the breast cancer is HER2-positive and hormone receptor-negative early-stage breast cancer. In some embodiments, the breast cancer is HER2-positive and hormone receptor-negative locally advanced breast cancer.

In some embodiments, the breast cancer has a clinical stage of T0 to T4 (e.g., T0, T1, T2, T3, or T4), N1 to N3 (e.g., N1, N2, or N3), and M0. In some embodiments, the breast cancer has a clinical stage of T2 to T4 (e.g., T2, T3, or T4), N0, and M0. The clinical stage is determined according to the AJCC.

In some embodiments, the subject with breast cancer is eligible for surgical treatment after receiving neoadjuvant therapy. In some embodiments, the subject with HER2-positive breast cancer is eligible for surgical treatment after receiving neoadjuvant therapy. In some embodiments, the subject with HER2-positive early-stage breast cancer is eligible for surgical treatment after receiving neoadjuvant therapy. In some embodiments, the subject with HER2-positive locally advanced breast cancer is eligible for surgical treatment after receiving neoadjuvant therapy.

In some embodiments, the subject with breast cancer is a subject who is eligible for surgical treatment after receiving neoadjuvant therapy. In some embodiments, the subject with HER2-positive breast cancer is a subject who is eligible for surgical treatment after receiving neoadjuvant therapy. In some embodiments, the subject with HER2-positive early-stage breast cancer is a subject who is eligible for surgical treatment after receiving neoadjuvant therapy. In some embodiments, the subject with HER2-positive locally advanced breast cancer is a subject who is eligible for surgical treatment after receiving neoadjuvant therapy.

In some embodiments, the subject with breast cancer has not previously received treatment for breast cancer. In some embodiments, the subject with breast cancer has not previously received systemic therapy for the treatment of breast cancer. In some embodiments, the subject with HER2-positive breast cancer has not previously received systemic therapy for the treatment of breast cancer. In some embodiments, the subject with HER2-positive early-stage breast cancer has not previously received systemic therapy for the treatment of breast cancer. In some embodiments, the subject with HER2-positive locally advanced breast cancer has not previously received systemic therapy for the treatment of breast cancer. In some embodiments, the subject with breast cancer has not previously received systemic therapy targeting breast cancer. In some embodiments, the subject with HER2-positive breast cancer has not previously received systemic therapy targeting breast cancer.

### Anti-HER2 Antibody-Drug Conjugate

The anti-HER2 antibody-drug conjugate used in the present disclosure is formed by linking a drug-linker having a structure represented by formula Ia below to an HER2-targeting antigen-binding construct, 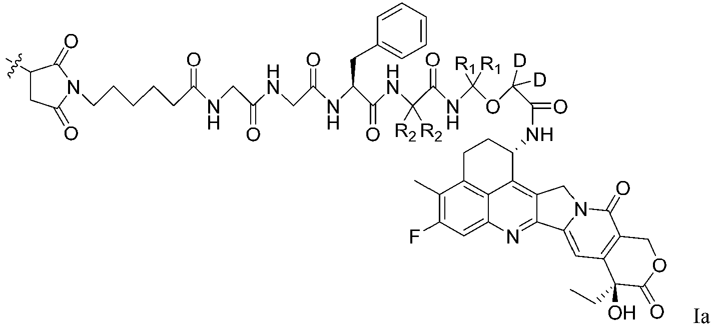 wherein
R₁ and R₂ in formula Ia are each independently selected from hydrogen (H) and deuterium (D), and position 3 of - (succinimidyl-3-yl-N)- in formula Ia (i.e., the position linked by " 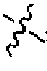 ") is linked to the HER2-targeting antigen-binding construct;
the HER2-targeting antigen-binding construct comprises a first antigen-binding fragment and a second antigen-binding fragment, wherein the first antigen-binding fragment comprises: a heavy chain CDR1 (HCDR1) comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, a light chain CDR1 (LCDR1) comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6; the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, position 3 of -(succinimidyl-3-yl-N)- is linked to the HER2-targeting antigen-binding construct via a thioether bond. In some embodiments, R₁ and R₂ are hydrogen.

In some embodiments, in the anti-HER2 antibody-drug conjugate, the average number of linked drug-linkers per HER2-targeting antigen-binding construct is 2 to 8. In some embodiments, in the anti-HER2 antibody-drug conjugate, the average number of linked drug-linkers per HER2-targeting antigen-binding construct is 4 to 7. In some embodiments, in the anti-HER2 antibody-drug conjugate, the average number of linked drug-linkers per HER2-targeting antigen-binding construct is 5 to 6. In some embodiments, in the anti-HER2 antibody-drug conjugate, the average number of linked drug-linkers per HER2-targeting antigen-binding construct is 5.5 to 6. In some embodiments, in the anti-HER2 antibody-drug conjugate, the average number of linked drug-linkers per HER2-targeting antigen-binding construct is 5.8 to 6.

**Table 1. CDR sequences of exemplary HER2-targeting antigen-binding construct**

| | | | |
|---|---|---|---|
| First antigen-binding fragment | HCDR1 | GFNIEDTYIH | SEQ ID NO: 1 |
| | HCDR2 | RIYPTNGYTRYADSVKG | SEQ ID NO: 2 |
| | HCDR3 | WGGDGFYAMDYW | SEQ ID NO: 3 |
| | LCDR1 | CRASQDVNTAVAW | SEQ ID NO: 4 |
| | LCDR2 | SASYLYS | SEQ ID NO: 5 |
| | LCDR3 | QQHYTTPPT | SEQ ID NO: 6 |
| Second antigen-binding fragment | HCDR1 | GFTFTDYTMD | SEQ ID NO: 9 |
| | HCDR2 | DVNPNSGGSIYNQRFKG | SEQ ID NO: 10 |
| | HCDR3 | NLGPSFYFDY | SEQ ID NO: 11 |
| | LCDR1 | KASQDVSIGVA | SEQ ID NO: 12 |
| | LCDR2 | SASYRYT | SEQ ID NO: 13 |
| | LCDR3 | QQYYIYPYT | SEQ ID NO: 14 |

It should be understood by those skilled in the art that, unless otherwise specified, the term "CDR" or "complementarity determining region" of a given antigen-binding fragment or a region thereof (e.g., a variable region) should be understood to encompass complementarity determining regions defined by any one of the known schemes. Although the CDRs claimed in the present disclosure are based on the sequences shown in Table 1 (one definition scheme), amino acid sequences corresponding to other CDR definition schemes (one of or a combination of several of the definition schemes well known in the art, such as AbM, CCG, Kabat, Chothia, IMGT, or Contact) should also fall within the protection scope of the present disclosure.

In some embodiments, the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the first antigen-binding fragment comprises a heavy chain variable region having the amino acid sequence set forth in SEQ ID NO: 7 and a light chain variable region having the amino acid sequence set forth in SEQ ID NO: 8. In some specific embodiments, the amino acid sequence of the heavy chain variable region of the first antigen-binding fragment is set forth in SEQ ID NO: 7, and the amino acid sequence of the light chain variable region of the first antigen-binding fragment is set forth in SEQ ID NO: 8.

In some embodiments, the first antigen-binding fragment comprises a heavy chain variable region and a light chain variable region, wherein the heavy chain variable region comprises an HCDR1 having the amino acid sequence set forth in SEQ ID NO: 1, an HCDR2 having the amino acid sequence set forth in SEQ ID NO: 2, and an HCDR3 having the amino acid sequence set forth in SEQ ID NO: 3, and the light chain variable region comprises an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 7, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 8.

EVQLVESGGGLVQPGGSLRLSCAASGFNIEDTYIHWVRQAPGKGLEWVARIYPTNGYTRYADSVKGRFT
ISADTSKNTAYLQMNSLRAEDTAVYYCSRWGGDGFYAMDYWGQGTLVTVSS (SEQ ID NO: 7);

DIQMTQSPSSLSASVGDRVTITCRASQDVNTAVAWYQQKPGKAPKLLIYSASYLYSGVPSRFSGSRSGTD
FTLTISSLQPEDFATYYCQQHYTTPPTFGQGTKVEIK (SEQ ID NO: 8).

In some embodiments, compared with the amino acid sequence set forth in SEQ ID NO: 7 or SEQ ID NO: 8, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions. In some embodiments, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. In some embodiments, the second antigen-binding fragment comprises a heavy chain variable region having the amino acid sequence set forth in SEQ ID NO: 15 and a light chain variable region having the amino acid sequence set forth in SEQ ID NO: 16. In some specific embodiments, the amino acid sequence of the heavy chain variable region of the second antigen-binding fragment is set forth in SEQ ID NO: 15, and the amino acid sequence of the light chain variable region of the second antigen-binding fragment is set forth in SEQ ID NO: 16.

In some embodiments, the second antigen-binding fragment comprises a heavy chain variable region and a light chain variable region, wherein the heavy chain variable region comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the light chain variable region comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 15, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 16.


 (SEQ ID NO: 15);


 (SEQ ID NO: 16).

In some embodiments, compared with the amino acid sequence set forth in SEQ ID NO: 15 or SEQ ID NO: 16, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in the FR regions.

In some embodiments, the HER2-targeting antigen-binding construct may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, and IgG4, or other types of immunoglobulins, preferably a heavy chain of IgG4. In some embodiments, the light chain constant region is derived from a human immunoglobulin light chain, for example, a κ light chain or a λ light chain of a human immunoglobulin. In some embodiments, the constant region may comprise any modification described in the text, for example, insertion, deletion, substitution, or chemical modification of amino acids. In some embodiments, lysine at the C-terminus of the heavy chain constant region may be present or deleted, and the deletion of lysine at the C-terminus of the heavy chain constant region generally occurs during recombinant expression. In some embodiments, the constant region comprises a mutation that alters the effector function. In some embodiments, any amino acid residue of the constant region may be substituted with an amino acid residue of any allotype.

In some embodiments, the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19. In some embodiments, the HER2-targeting antigen-binding construct comprises a first polypeptide chain having the amino acid sequence set forth in SEQ ID NO: 17, a second polypeptide chain having the amino acid sequence set forth in SEQ ID NO: 18, and a third polypeptide chain having the amino acid sequence set forth in SEQ ID NO: 19. In some specific embodiments, the HER2-targeting antigen-binding construct consists of three polypeptide chains, wherein the amino acid sequence of a first polypeptide chain is set forth in SEQ ID NO: 17, the amino acid sequence of a second polypeptide chain is set forth in SEQ ID NO: 18, and the amino acid sequence of a third polypeptide chain is set forth in SEQ ID NO: 19.

In some embodiments, the HER2-targeting antigen-binding construct comprises a first polypeptide chain, a second polypeptide chain, and a third polypeptide chain, wherein the first polypeptide chain comprises an HCDR1 having the amino acid sequence set forth in SEQ ID NO: 1, an HCDR2 having the amino acid sequence set forth in SEQ ID NO: 2, an HCDR3 having the amino acid sequence set forth in SEQ ID NO: 3, an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6, the second polypeptide chain comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the third polypeptide chain comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the first polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, the second polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and the third polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

EVQLVESGGGLVQPGGSLRLSCAASGFNIEDTYIHWVRQAPGKGLEWVARIYPTNGYTRYADSVKGRFT
ISADTSKNTAYLQMNSLRAEDTAVYYCSRWGGDGFYAMDYWGQGTLVTVSSGGGGSGGGGSGGGGS
GGGGSDIQMTQSPSSLSASVGDRVTITCRASQDVNTAVAWYQQKPGKAPKLLIYSASYLYSGVPSRFSGS
RSGTDFTLTISSLQPEDFATYYCQQHYTTPPTFGQGTKVEIKGEPKSSDKTHTCPPCPAPELLGGPSVFLFP
PKPKDTLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHNHYTQKSLSLSPGK
(SEQ ID NO: 17);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVCTLPPSREEMTKNQVSLSCAVKGFYPSDIAVEWESNGQPENNYKTTPPVLDSDGSFFLVSKLTVDK
SRWQQGNVFSCSVMHEALHNRFTQKSLSLSPGK (SEQ ID NO: 18);

DIQMTQSPSSLSASVGDRVTITCKASQDVSIGVAWYQQKPGKAPKLLIYSASYRYTGVPSRFSGSGSGTDF
TLTISSLQPEDFATYYCQQYYIYPYTFGQGTKVEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNR
GEC (SEQ ID NO: 19).

In some embodiments, lysine at the C-terminus of the amino acid sequence set forth in SEQ ID NO: 17 is deleted, as set forth in SEQ ID NO: 20. In some embodiments, lysine at the C-terminus of the amino acid sequence set forth in SEQ ID NO: 18 is deleted, as set forth in SEQ ID NO: 21. In some embodiments, lysine at the C-terminus of each of the first polypeptide chain and the second polypeptide chain is deleted; the amino acid sequence of the first polypeptide chain is set forth in SEQ ID NO: 20, and the amino acid sequence of the second polypeptide chain is set forth in SEQ ID NO: 21.

EVQLVESGGGLVQPGGSLRLSCAASGFNIEDTYIHWVRQAPGKGLEWVARIYPTNGYTRYADSVKGRFT
ISADTSKNTAYLQMNSLRAEDTAVYYCSRWGGDGFYAMDYWGQGTLVTVSSGGGGSGGGGSGGGGS
GGGGSDIQMTQSPSSLSASVGDRVTITCRASQDVNTAVAWYQQKPGKAPKLLIYSASYLYSGVPSRFSGS
RSGTDFTLTISSLQPEDFATYYCQQHYTTPPTFGQGTKVEIKGEPKSSDKTHTCPPCPAPELLGGPSVFLFP
PKPKDTLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHNHYTQKSLSLSPG (SEQ
ID NO: 20);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVCTLPPSREEMTKNQVSLSCAVKGFYPSDIAVEWESNGQPENNYKTTPPVLDSDGSFFLVSKLTVDK
SRWQQGNVFSCSVMHEALHNRFTQKSLSLSPG (SEQ ID NO: 21).

In some embodiments, compared with the amino acid sequence set forth in SEQ ID NO: 17, 18, 19, 20, or 21, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions or constant regions.

In some other embodiments, the HER2-targeting antigen-binding construct is selected from Expi Her2-1, Expi Her2-3, Expi Her2-4, Expi Her2-5, 23C2 Her2-1, 23C2 Her2-3, 23C2 Her2-4, and 23C2 Her2-5 (see WO2021219046 or CN115279791A). In some other embodiments, the HER2-targeting antigen-binding construct is selected from zanidatamab (ZW25), KN026, MBS301, KM257, and BCD-147.

The anti-HER2 antibody-drug conjugate used in the present disclosure may also be represented by the structure represented by formula II below: 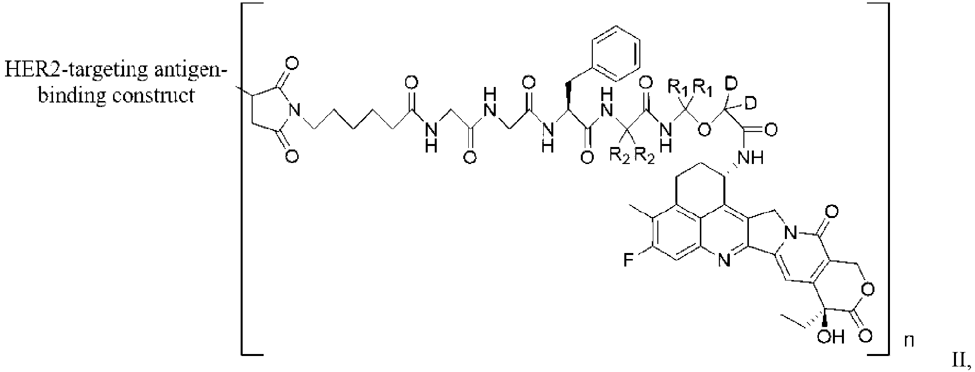 wherein R₁ and R₂ in formula II are each independently selected from hydrogen and deuterium, and the HER2-targeting antigen-binding construct is as described above. In some embodiments, the drug-linker is linked to the HER2-targeting antigen-binding construct via a thioether bond (position 3 of -(succinimidyl-3-yl-N)-). In some embodiments, R₁ and R₂ are hydrogen. n has the same meaning as DAR and represents the average number of linked cytotoxic drugs per HER2-targeting antigen-binding construct. In some embodiments, n is 2 to 8. In some embodiments, n is 4 to 7. In some embodiments, n is 5 to 6. In some embodiments, n is 5.5 to 6. In some embodiments, n is 5.8 to 6.

The antibody-drug conjugate preferably used in the present disclosure is formed by linking a drug-linker having a structure represented by formula Ia below to an HER2-targeting antigen-binding construct, 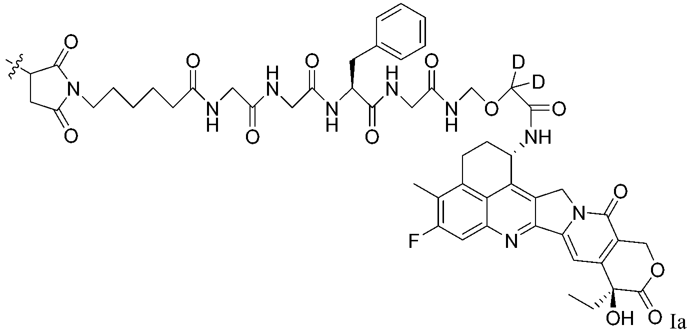 wherein
position 3 of -(succinimidyl-3-yl-N)- in formula Ia is linked to the HER2-targeting antigen-binding construct;
the HER2-targeting antigen-binding construct consists of three polypeptide chains, wherein the amino acid sequence of a first polypeptide chain is set forth in SEQ ID NO: 17 or is a variant thereof with a deletion of lysine at the C-terminus, the amino acid sequence of a second polypeptide chain is set forth in SEQ ID NO: 18 or is a variant thereof with a deletion of lysine at the C-terminus, and the amino acid sequence of a third polypeptide chain is set forth in SEQ ID NO: 19 (for example, the first polypeptide chain has the amino acid sequence set forth in SEQ ID NO: 17, the second polypeptide chain has the amino acid sequence set forth in SEQ ID NO: 18, and the third polypeptide chain has the amino acid sequence set forth in SEQ ID NO: 19; or, the first polypeptide chain has the amino acid sequence set forth in SEQ ID NO: 20, the second polypeptide chain has the amino acid sequence set forth in SEQ ID NO: 21, and the third polypeptide chain has the amino acid sequence set forth in SEQ ID NO: 19; or, the first polypeptide chain has the amino acid sequence set forth in SEQ ID NO: 20, the second polypeptide chain has the amino acid sequence set forth in SEQ ID NO: 18, and the third polypeptide chain has the amino acid sequence set forth in SEQ ID NO: 19; or, the first polypeptide chain has the amino acid sequence set forth in SEQ ID NO: 17, the second polypeptide chain has the amino acid sequence set forth in SEQ ID NO: 21, and the third polypeptide chain has the amino acid sequence set forth in SEQ ID NO: 19); and
the average number of linked drug-linkers for one HER2-targeting antigen-binding construct is 5 to 6.

In some embodiments, position 3 of -(succinimidyl-3-yl-N)- in formula Ia is linked to the HER2-targeting antigen-binding construct via a thioether bond.

The antibody-drug conjugate preferably used in the present disclosure may also be represented by the structure represented by formula II below: 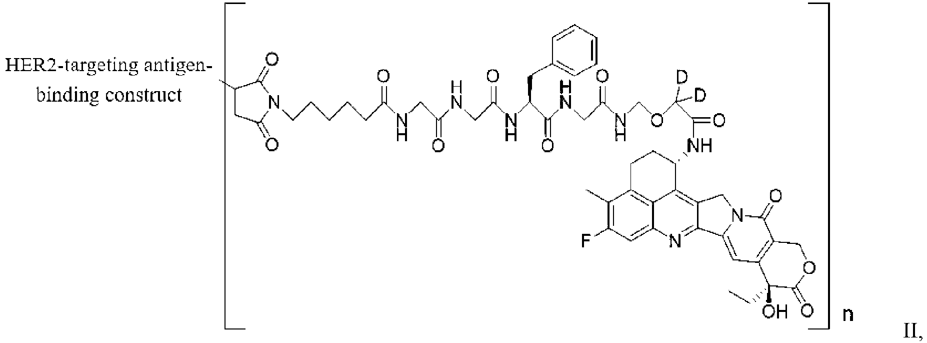 wherein n is 5 to 6;
the HER2-targeting antigen-binding construct consists of three polypeptide chains, wherein the amino acid sequence of a first polypeptide chain is set forth in SEQ ID NO: 17 or is a variant thereof with a deletion of lysine at the C-terminus, the amino acid sequence of a second polypeptide chain is set forth in SEQ ID NO: 18 or is a variant thereof with a deletion of lysine at the C-terminus, and the amino acid sequence of a third polypeptide chain is set forth in SEQ ID NO: 19 (for example, the first polypeptide chain has the amino acid sequence set forth in SEQ ID NO: 17, the second polypeptide chain has the amino acid sequence set forth in SEQ ID NO: 18, and the third polypeptide chain has the amino acid sequence set forth in SEQ ID NO: 19; or, the first polypeptide chain has the amino acid sequence set forth in SEQ ID NO: 20, the second polypeptide chain has the amino acid sequence set forth in SEQ ID NO: 21, and the third polypeptide chain has the amino acid sequence set forth in SEQ ID NO: 19; or, the first polypeptide chain has the amino acid sequence set forth in SEQ ID NO: 20, the second polypeptide chain has the amino acid sequence set forth in SEQ ID NO: 18, and the third polypeptide chain has the amino acid sequence set forth in SEQ ID NO: 19; or, the first polypeptide chain has the amino acid sequence set forth in SEQ ID NO: 17, the second polypeptide chain has the amino acid sequence set forth in SEQ ID NO: 21, and the third polypeptide chain has the amino acid sequence set forth in SEQ ID NO: 19). In some embodiments, the drug-linker is linked to the HER2-targeting antigen-binding construct via a thioether bond.

In some other specific embodiments, the anti-HER2 antibody-drug conjugate of the present disclosure is selected from Kadcyla^{®}, Enhertu^{®}, Aidixi^{®}, MRG002, ARX788, A166, SHR-A1811, BB-1701, SYD985, FS-1502, and BAT8001.

The anti-HER2 antibody-drug conjugate described in the present disclosure also includes isomers or pharmaceutically acceptable salts of the anti-HER2 antibody-drug conjugate, or solvates of the anti-HER2 antibody-drug conjugate, isomers thereof, or pharmaceutically acceptable salts thereof.

### Technical Effects

Administration of the anti-HER2 antibody-drug conjugate of the present disclosure achieves one or more of the following effects:
(1) providing benefit to a subject with breast cancer, wherein the breast cancer is preferably early-stage breast cancer or locally advanced breast cancer;
(2) exhibiting good safety;
(3) being well-tolerated in subjects; and
(4) exhibiting good efficacy in neoadjuvant therapy of HER2-positive breast cancer.

### Definitions and Description

Unless otherwise stated, the following terms used in the present disclosure shall have the following meanings. A certain term, unless otherwise specifically defined, should not be considered uncertain or unclear, but interpreted according to its common meaning in the art. When referring to a trade name in the present disclosure, it is intended to refer to its corresponding commercial product or its active ingredient.

As used herein, the structure of "-(succinimidyl-3-yl-N)-" is as the formula below:

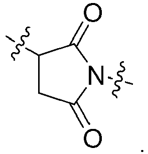

Unless otherwise stated, the absolute configuration of a stereogenic center is represented by a wedged solid bond ( 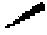 ) and a wedged dashed bond ( 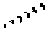 ).

Unless otherwise specified, when a certain group has a linkable site, the linkage between the site and another group may be represented by a wavy line ( 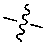 ).

The term "antigen-binding construct" refers to any agent capable of binding to an antigen, such as a polypeptide or a polypeptide complex. In some aspects, the antigen-binding construct is a polypeptide that specifically binds to a target antigen. The antigen-binding construct may be a monomer, a dimer, a polymer, a protein, a peptide, a protein or peptide complex, an antibody or an antigen-binding fragment thereof, and the like. The antigen-binding construct may be a monospecific, bispecific, or multispecific polypeptide construct. In some aspects, the antigen-binding construct may include, for example, one or more antigen-binding fragments (e.g., Fab or scFv) linked to one or more Fc.

The "antigen-binding fragment" of an antibody refers to one or more fragments of the antibody that retain the functionality of specifically binding to an antigen (e.g., HER2 protein). It has been demonstrated that the antigen-binding functionality of an antibody can be implemented by fragments of a full-length antibody. Examples encompassed within the term "antigen-binding fragment" of an antibody include: (i) a Fab fragment: a monovalent fragment consisting of VL, VH, CL, and CH1 domains; (ii) a F(ab')₂ fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); and (vi) a nanobody, an antibody comprising a single variable domain and two constant domains. Furthermore, although the two domains VL and VH of the Fv fragment are encoded by different genes, the VH and VL can be joined via a linker by recombinant methods into a single protein chain in which the VL and VH are paired to form a monovalent molecule referred to as a single-chain Fv (scFv) (see, Bird et al., Science. 242:423-426 (1988); Huston et al., Proc. Natl. Acad. Sci. 85:5879-5883 (1988)). These single-chain antibodies are also encompassed within the term "antigen-binding fragment". These antibody fragments can be obtained using conventional techniques known to those skilled in the art, and the fragments can be subjected to functional screening using the same method as full-length antibodies.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to acquire the maximum alignment for the full length of sequences being compared.

The term "treating" refers to administering the compound described in the present disclosure to prevent, ameliorate, or eliminate a disease or one or more symptoms associated with the disease, and includes, but is not limited to:
(i) preventing the occurrence of a disease or disease state in a mammal, particularly when such a mammal is predisposed to the disease state but has not yet been diagnosed as having it;
(ii) inhibiting a disease or disease state, i.e., arresting its progression;
(iii) alleviating a disease or disease state, i.e., causing its regression; and
(iv) reducing any direct or indirect pathological consequences of a disease or disease state.

The term "therapeutically effective amount" refers to an amount of the compound of the present disclosure for (i) treating or preventing a specific disease, condition, or disorder, (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure that constitutes a "therapeutically effective amount" may vary depending on factors such as the compound and its ability to elicit a desired response in an individual, the disease state and its severity, the mode of administration, and the age, sex, and body weight of the mammal to be treated.

"Neoadjuvant therapy" refers to a therapy in which a therapeutic agent is administered to a subject prior to surgery. The term "administer", "administration", or "administering" refers to physically introducing a therapeutic agent to a subject using any one of a variety of methods and delivery systems known to those skilled in the art.

Routes of administration for antibody-drug conjugates (e.g., anti-HER2 antibody-drug conjugates) include intravenous, intramuscular, intraperitoneal, spinal, or other parenteral routes of administration. As used herein, the term "parenteral administration" refers to modes of administration other than enteral administration, typically performed by injection, including, but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion, as well as *in vivo* electroporation. The administration may also be performed, for example, once, multiple times, and/or over one or more extended periods of time.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of a compound (e.g., the antibody-drug conjugate of the present disclosure) that exhibits safety and efficacy when used in mammals and possesses the desired biological activity; for example, such a salt may be a metal salt, an ammonium salt, a salt formed with an organic base, a salt formed with an inorganic acid, a salt formed with an organic acid, a salt formed with a basic or acidic amino acid, or the like.

The term "excipient" refers to any ingredient other than the active ingredient (e.g., the antibody-drug conjugate of the present disclosure). The selection of the excipient will largely depend on factors such as the particular mode of administration, the effect of the excipient on solubility and stability, and the nature of the dosage form.

The term "solvate" refers to a substance formed by the association of a compound with a solvent molecule.

In the present disclosure, "HER2-positive" breast cancer is not particularly limited as long as it is recognized by those skilled in the art as breast cancer with HER2 overexpression, and preferred examples include breast cancer determined to have HER2 expression of 3+ by immunohistochemistry (IHC) (i.e., IHC 3+), or/and breast cancer determined to be positive for HER2 expression by ISH (i.e., ISH+). It should be noted that the in situ hybridization of the present application includes fluorescence in situ hybridization (FISH) and dual-color in situ hybridization (DISH).

Herein, the terms "subject", "patient", and "entity" can be used interchangeably. The "subject", "patient", or "entity" includes any human or non-human animal. The term "non-human animal" includes, but is not limited to, vertebrates such as non-human primates, sheep, dogs, and rodents such as mice, rats, and guinea pigs. In some embodiments, the subject, patient, or entity is a mammal. In some embodiments, the subject, patient, or entity is a mouse. In some embodiments, the subject, patient, or entity is a human.

The term "pharmaceutical composition" refers to a mixture composed of one or more active ingredients (e.g., the anti-HER2 antibody-drug conjugate of the present disclosure) and pharmaceutically acceptable excipients. The pharmaceutical composition is intended to facilitate the administration of the active ingredient(s) to a subject. Herein, the terms "pharmaceutical composition" and "formulation" have the same meaning and can be used interchangeably. The word "comprise" and variations thereof such as "comprises" or "comprising" should be understood in an openended and non-exclusive sense, i.e., "including but not limited to".

Herein, unless otherwise specified clearly in the context, singular terms encompass plural referents, and vice versa. As used herein, "about" means being within an acceptable error range determined by those of ordinary skill in the art for a specific value, which will depend in part on how the value is measured or determined, i.e., the limitation by the measurement system. For example, "about" may mean being within 1× or more than 1× standard deviation, as practiced in the art. Alternatively, "about" may mean a range of up to ±5%, for example, fluctuating within a specific numerical range given ±2%, ±1%, or ±0.5%. Where a specific value is given in the present disclosure or in the claims, unless otherwise stated, "about" should be considered to mean being within an acceptable error range for that specific value. Herein, unless otherwise stated, all values for drug doses, timing, step parameters, or conditions are modified by "about" by default.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

The present disclosure further provides the following specific embodiments, which, however, are not intended to limit the protection scope of the present disclosure:
Embodiment 1. A method for neoadjuvant therapy of breast cancer in a subject, comprising administering to the subject an anti-HER2 antibody-drug conjugate, wherein the anti-HER2 antibody-drug conjugate is formed by linking a drug-linker having a structure represented by formula Ia below 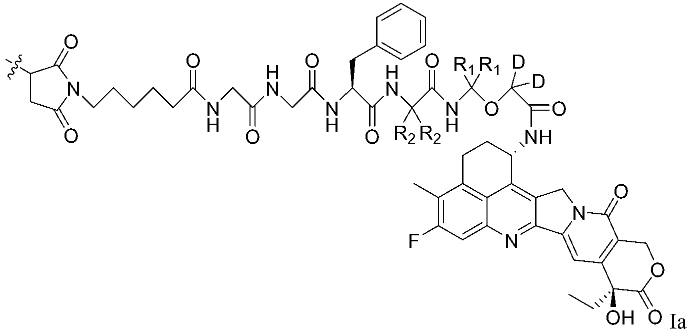
   to an HER2-targeting antigen-binding construct, wherein
   R₁ and R₂ in formula Ia are each independently selected from hydrogen and deuterium; preferably, R₁ and R₂ are hydrogen;
   position 3 of -(succinimidyl-3-yl-N)- is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 2. The method according to embodiment 1, wherein an average number of linked drug-linkers per HER2-targeting antigen-binding construct is 4 to 7.
Embodiment 3. The method according to embodiment 2, wherein the average number of linked drug-linkers per HER2-targeting antigen-binding construct is 5 to 6.
Embodiment 4. The method according to embodiment 3, wherein the average number of linked drug-linkers per HER2-targeting antigen-binding construct is 5.5 to 6.
Embodiment 5. The method according to any one of embodiments 1-4, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 6. The method according to any one of embodiments 1-5, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 7. The method according to any one of embodiments 1-6, wherein the anti-HER2 antibody-drug conjugate is administered in a therapeutically effective amount.
Embodiment 8. The method according to any one of embodiments 1-7, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 9. The method according to embodiment 8, wherein the anti-HER2 antibody-drug conjugate is administered at a dose of 1.5 mg/kg, 3 mg/kg, 4.5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 10. The method according to any one of embodiments 1-9, wherein one treatment cycle consists of 1 week, 2 weeks, 3 weeks, or 4 weeks, and the anti-HER2 antibody-drug conjugate is administered once per treatment cycle; preferably, one treatment cycle consists of 3 weeks, and the anti-HER2 antibody-drug conjugate is administered once per treatment cycle.
Embodiment 11. The method according to any one of embodiments 1-10, wherein the anti-HER2 antibody-drug conjugate is administered for a total of 6-8 treatment cycles; preferably, the anti-HER2 antibody-drug conjugate is administered continuously or intermittently for 6-8 treatment cycles, preferably for a total of 6 treatment cycles or 8 treatment cycles.
Embodiment 12. The method according to any one of embodiments 1-11, wherein the anti-HER2 antibody-drug conjugate is formulated into a formulation for intravenous injection or infusion.
Embodiment 13. The method according to any one of embodiments 1-12, wherein the anti-HER2 antibody-drug conjugate is administered by intravenous infusion.
Embodiment 14. The method according to any one of embodiments 1-13, wherein the breast cancer is early-stage or locally advanced breast cancer.
Embodiment 15. The method according to any one of embodiments 1-14, wherein the breast cancer is invasive breast cancer, preferably unilateral invasive breast cancer.
Embodiment 16. The method according to any one of embodiments 1-15, wherein the breast cancer is HER2-expressing breast cancer.
Embodiment 17. The method according to any one of embodiments 1-16, wherein the breast cancer is HER2-positive breast cancer.
Embodiment 18. The method according to embodiment 17, wherein the HER2-positive breast cancer is breast cancer determined to have HER2 expression of 3+ by IHC or/and breast cancer determined to be positive for HER2 expression by ISH.
Embodiment 19. The method according to embodiment 17 or 18, wherein the HER2-positive breast cancer is breast cancer determined to have HER2 expression of 2+ by IHC and determined to be positive for HER2 expression by ISH.
Embodiment 20. The method according to any one of embodiments 1-19, wherein the breast cancer is hormone receptor-positive breast cancer.
Embodiment 21. The method according to embodiment 20, wherein the breast cancer is estrogen receptor- or/and progesterone receptor-positive breast cancer.
Embodiment 22. The method according to any one of embodiments 1-19, wherein the breast cancer is hormone receptor-negative breast cancer.
Embodiment 23. The method according to embodiment 22, wherein the breast cancer is estrogen receptor- or/and progesterone receptor-negative breast cancer.
Embodiment 24. The method according to any one of embodiments 1-13, wherein the breast cancer is HER2-positive early-stage or locally advanced breast cancer.
Embodiment 25. The method according to any one of embodiments 1-13, wherein the breast cancer is HER2-positive and hormone receptor-positive early-stage or locally advanced breast cancer.
Embodiment 26. The method according to any one of embodiments 1-13, wherein the breast cancer is HER2-positive and hormone receptor-negative early-stage or locally advanced breast cancer.
Embodiment 27. The method according to any one of embodiments 1-26, wherein the breast cancer has a clinical stage of T0 to T4, N1 to N3, and M0.
Embodiment 28. The method according to any one of embodiments 1-26, wherein the breast cancer has a clinical stage of T2 to T4, N0, and M0.
Embodiment 29. The method according to any one of embodiments 1-28, wherein the subject with breast cancer is eligible for surgical treatment after receiving neoadjuvant therapy.
Embodiment 30. The method according to any one of embodiments 1-29, wherein the subject with breast cancer has not previously received treatment for breast cancer.
Embodiment 31. The method according to embodiment 30, wherein the subject with breast cancer has not previously received systemic therapy for the treatment of breast cancer.
Embodiment 32. Use of the anti-HER2 antibody-drug conjugate for preparing a medicament for neoadjuvant therapy of breast cancer, wherein the anti-HER2 antibody-drug conjugate is formed by linking a drug-linker having a structure represented by formula Ia below 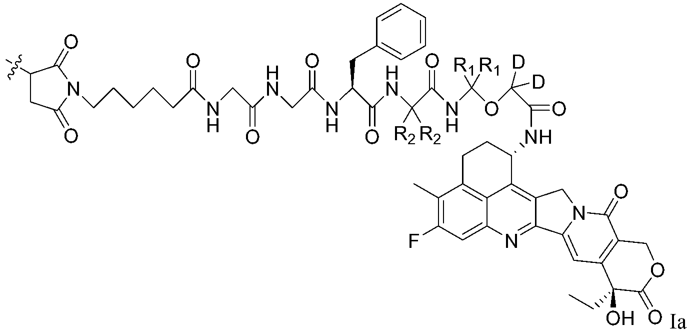
   to an HER2-targeting antigen-binding construct, wherein
   R₁ and R₂ in formula Ia are each independently selected from hydrogen and deuterium; preferably, R₁ and R₂ are hydrogen;
   position 3 of -(succinimidyl-3-yl-N)- is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 33. The use according to embodiment 32, wherein an average number of linked drug-linkers per HER2-targeting antigen-binding construct is 4 to 7.
Embodiment 34. The use according to embodiment 33, wherein the average number of linked drug-linkers per HER2-targeting antigen-binding construct is 5 to 6.
Embodiment 35. The use according to embodiment 34, wherein the average number of linked drug-linkers per HER2-targeting antigen-binding construct is 5.5 to 6.
Embodiment 36. The use according to any one of embodiments 32-35, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 37. The use according to any one of embodiments 32-36, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 38. The use according to any one of embodiments 32-37, wherein the anti-HER2 antibody-drug conjugate is administered in a therapeutically effective amount.
Embodiment 39. The use according to any one of embodiments 32-37, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 40. The use according to embodiment 39, wherein the anti-HER2 antibody-drug conjugate is administered at a dose of 1.5 mg/kg, 3 mg/kg, 4.5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 41. The use according to any one of embodiments 32-40, wherein one treatment cycle consists of 1 week, 2 weeks, 3 weeks, or 4 weeks, and the anti-HER2 antibody-drug conjugate is administered once per treatment cycle; preferably, one treatment cycle consists of 3 weeks, and the anti-HER2 antibody-drug conjugate is administered once per treatment cycle.
Embodiment 42. The use according to any one of embodiments 32-41, wherein the anti-HER2 antibody-drug conjugate is administered for a total of 6-8 treatment cycles; preferably, the anti-HER2 antibody-drug conjugate is administered continuously or intermittently for 6-8 treatment cycles, preferably for a total of 6 treatment cycles or 8 treatment cycles.
Embodiment 43. The use according to any one of embodiments 32-42, wherein the anti-HER2 antibody-drug conjugate is formulated into a formulation for intravenous injection or infusion.
Embodiment 44. The use according to any one of embodiments 32-43, wherein the anti-HER2 antibody-drug conjugate is administered by intravenous infusion.
Embodiment 45. The use according to any one of embodiments 32-44, wherein the breast cancer is early-stage or locally advanced breast cancer.
Embodiment 46. The use according to any one of embodiments 32-45, wherein the breast cancer is invasive breast cancer, preferably unilateral invasive breast cancer.
Embodiment 47. The use according to any one of embodiments 32-46, wherein the breast cancer is HER2-expressing breast cancer.
Embodiment 48. The use according to any one of embodiments 32-47, wherein the breast cancer is HER2-positive breast cancer.
Embodiment 49. The use according to embodiment 48, wherein the HER2-positive breast cancer is breast cancer determined to have HER2 expression of 3+ by IHC or/and breast cancer determined to be positive for HER2 expression by ISH.
Embodiment 50. The use according to embodiment 48 or 49, wherein the HER2-positive breast cancer is breast cancer determined to have HER2 expression of 2+ by IHC and determined to be positive for HER2 expression by ISH.
Embodiment 51. The use according to any one of embodiments 32-50, wherein the breast cancer is hormone receptor-positive breast cancer.
Embodiment 52. The use according to embodiment 51, wherein the breast cancer is estrogen receptor- or/and progesterone receptor-positive breast cancer.
Embodiment 53. The use according to any one of embodiments 32-50, wherein the breast cancer is hormone receptor-negative breast cancer.
Embodiment 54. The use according to embodiment 53, wherein the breast cancer is estrogen receptor- or/and progesterone receptor-negative breast cancer.
Embodiment 55. The use according to any one of embodiments 32-44, wherein the breast cancer is HER2-positive early-stage or locally advanced breast cancer.
Embodiment 56. The use according to any one of embodiments 32-44, wherein the breast cancer is HER2-positive and hormone receptor-positive early-stage or locally advanced breast cancer.
Embodiment 57. The use according to any one of embodiments 32-44, wherein the breast cancer is HER2-positive and hormone receptor-negative early-stage or locally advanced breast cancer.
Embodiment 58. The use according to any one of embodiments 32-57, wherein the breast cancer has a clinical stage of T0 to T4, N1 to N3, and M0.
Embodiment 59. The use according to any one of embodiments 32-57, wherein the breast cancer has a clinical stage of T2 to T4, N0, and M0.
Embodiment 60. The use according to any one of embodiments 32-59, wherein the subject with breast cancer is a subject who is eligible for surgical treatment after receiving neoadjuvant therapy.
Embodiment 61. The use according to any one of embodiments 32-60, wherein the subject with breast cancer has not previously received treatment for breast cancer.
Embodiment 62. The use according to embodiment 61, wherein the subject with breast cancer has not previously received systemic therapy for the treatment of breast cancer.

### Example

For clarity, the present disclosure is further described with the following examples, which are, however, not intended to limit the scope of the present disclosure.

The content of the patent application WO2022033578 or CN115702008A is incorporated herein by reference in its entirety. In the following examples, the anti-HER2 antibody-drug conjugate is prepared according to the preparation method described in WO2022033578 to obtain the anti-HER2 antibody-drug conjugate represented by the formula below (hereinafter referred to as ADC1): 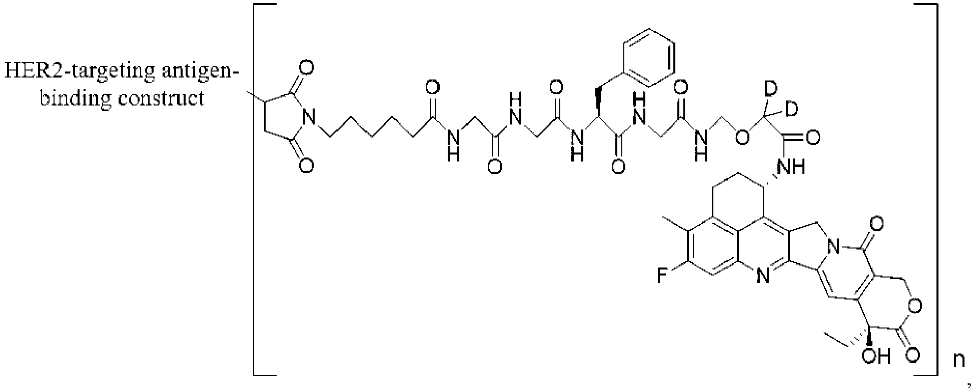 wherein the drug-linker is linked to the HER2-targeting antigen-binding construct via a thioether bond, and n is 5 to 6. Briefly, the nucleic acid sequences encoding the three polypeptide chains of the HER2-targeting antigen-binding construct (with amino acid sequences set forth in SEQ ID NOs: 17, 18, and 19, or set forth in SEQ ID NOs: 20, 21, and 19, respectively) are each cloned into a pcDNA3.1 expression vector, and the vectors are co-transfected into FUT8-knockout CHO-S cells for expression. The HER2-targeting antigen-binding construct is then prepared after purification via Protein A. The HER2-targeting antigen-binding construct is treated with tris(2-carboxyethyl)phosphine hydrochloride and subsequently reacted with a linker-payload selected from the structure represented by the formula below to finally obtain ADC1:

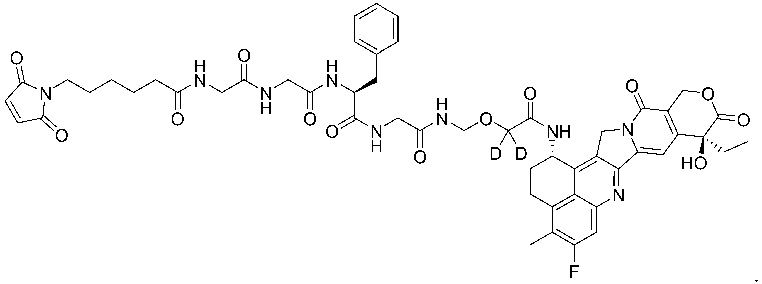

### Example 1: Clinical Trial of HER2-Expressing Breast Cancer

### 1. Study objectives:

### 1.1 Primary objective:

To evaluate the efficacy of ADC1 in neoadjuvant therapy of HER2-positive breast cancer.

### 1.2 Secondary objective:

To evaluate the safety and immunogenicity of ADC1 in neoadjuvant therapy of HER2-positive breast cancer;

### 2. Test subjects: subjects with early-stage or locally advanced HER2-positive breast cancer

### 3. Inclusion criteria

Subjects who meet all of the following inclusion criteria can be enrolled in this trial:
(1) Subjects voluntarily participated in this study, signed the informed consent form, and demonstrated good compliance;
(2) Age: 18-75 years old (at the time of signing the informed consent form); ECOG score: 0 to 1; expected survival time of more than 3 months;
(3) Subjects with histopathologically confirmed invasive breast cancer who had not previously received any systemic anti-tumor therapy for breast cancer;
(4) Confirmed HER2-positive status (with reference to the 2018 ASCO/CAP HER2 testing guidelines, where HER2-positive is defined as an immunohistochemistry result of 3+ or a positive FISH dual-probe result);
(5) Confirmed HR-positive or HR-negative status (with reference to the 2020 ASCO/CAP guidelines, where HR-positive includes ER-positive and/or PR-positive, i.e., the proportion of positively stained tumor cells among all tumor cells is ≥1%);
(6) Clinical stage at the time of visit being T0-4, N1-3, and M0, or being T2-4, N0, and M0 (determined according to the AJCC 8th edition breast cancer staging system);
(7) Subjects agreed to receive breast cancer resection when the surgical criteria were met after neoadjuvant therapy;
(8) Good function of major organs, meeting the criteria for blood routine examination, biochemical examination, coagulation function examination, and cardiac color ultrasound assessment;
(9) Female subjects of childbearing age should agree to take necessary contraceptive measures (such as intrauterine devices, contraceptives, or condoms) during the study and for 6 months after the study; the serum pregnancy test results should be negative within 7 days before enrollment, and the subjects must be in the non-lactation period; male subjects should agree to take necessary contraceptive measures during the study and for 6 months after the study.

### 4. Test drug

ADC1 for injection (specification: 100 mg/vial), developed and provided by Chia Tai Tianqing Pharmaceutical Group Nanjing Shunxin Pharmaceutical Co., Ltd.

### 5. Treatment regimen

ADC1 was administered by intravenous infusion once every 3 weeks, with every 3 weeks (21 days) defined as one treatment cycle. Cohort 1 received administration at a dose of 6 mg/kg for 6 treatment cycles; Cohort 2 received administration at a dose of 6 mg/kg for 8 treatment cycles; Cohort 3 received administration at a dose of 7.5 mg/kg for 6 treatment cycles; Cohort 4 received administration at a dose of 7.5 mg/kg for 8 treatment cycles. The number of subjects enrolled in each cohort was 26.

The administration regimens of the four cohorts could be adjusted according to the severity of the disease, the response to the disease, any treatment-related toxicity, and the age and health status of a patient.

### 6. Evaluation criteria

Efficacy evaluation: the disease status was determined using the RECIST 1.1 criteria.

Safety evaluation: the severity of adverse events was determined using the NCI-CTC AE 5.0 criteria.

### 7. Endpoint indicators

Primary endpoint: total pathological complete response (tpCR) rate.

Secondary endpoints: breast pathological complete response (bpCR) rate, objective response rate (ORR), event-free survival (EFS), invasive disease-free survival (IDFS), overall survival (OS), and the like.

### 8. Results

### 8.1 ORR (preoperative assessment):

ORR was calculated for subjects in Cohorts 1-4 who completed or were undergoing neoadjuvant therapy.

The ORR of the 6 mg/kg groups (Cohort 1 + Cohort 2) was 100% (52/52). The data of the 7.5 mg/kg groups are still being analyzed, and their ORR results are expected to be substantially comparable to those of the 6 mg/kg groups.

### 8.2 tpCR (postoperative evaluation):

As of the clinical data cutoff date, the number of subjects achieving tpCR was recorded and the tpCR rate was calculated for evaluable cases (because some patients are still in the neoadjuvant therapy phase and have not yet undergone breast cancer resection, the total number of evaluable cases in each cohort is lower than the number of enrolled patients:

| | | Total number of evaluable cases | Number of cases achieving tpCR |
|---|---|---|---|
| 6 mg/kg dose group | Cohort 1 (6 cycles of administration) | 24 | 14 |
| | Cohort 2 (8 cycles of administration) | 15 | 12 |
| 7.5 mg/kg dose group | Cohort 3 (6 cycles of administration) | 3 | 1 |

As can be seen from the above table, patients in Cohort 1 administered at a dose of 6 mg/kg for 6 cycles could achieve a tpCR rate of about 58.3%; patients in Cohort 2 administered at a dose of 6 mg/kg for 8 cycles could achieve a tpCR rate of 80%.

The results indicated that ADC1 can exhibit good efficacy in neoadjuvant therapy of early-stage or locally advanced HER2-positive breast cancer. Meanwhile, ADC1 can demonstrate good safety in clinical use.

## Claims

1. An anti-HER2 antibody-drug conjugate for use in neoadjuvant therapy of breast cancer in a subject, wherein the anti-HER2 antibody-drug conjugate is formed by linking a drug-linker having a structure represented by formula Ia below 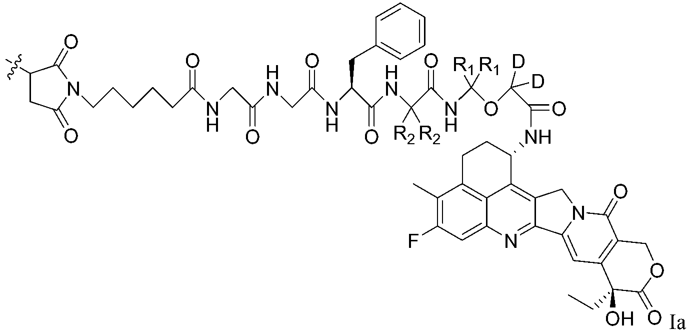
to an HER2-targeting antigen-binding construct, wherein
R₁ and R₂ in formula Ia are each independently selected from hydrogen and deuterium; preferably, R₁ and R₂ are hydrogen;
position 3 of -(succinimidyl-3-yl-N)- is linked to the HER2-targeting antigen-binding construct;
the HER2-targeting antigen-binding construct comprises a first antigen-binding fragment and a second antigen-binding fragment;
the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

2. The anti-HER2 antibody-drug conjugate for use in neoadjuvant therapy of breast cancer in a subject according to claim 1, wherein an average number of linked drug-linkers per HER2-targeting antigen-binding construct is 4 to 7, 5 to 6, or 5.5 to 6.

3. The anti-HER2 antibody-drug conjugate for use in neoadjuvant therapy of breast cancer in a subject according to claim 1 or 2, wherein
(i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
(ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
(v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
(vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
(vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and
the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.

4. The anti-HER2 antibody-drug conjugate for use in neoadjuvant therapy of breast cancer in a subject according to any one of claims 1-3, wherein
(i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
(ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
(iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
(viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

5. The anti-HER2 antibody-drug conjugate for use in neoadjuvant therapy of breast cancer in a subject according to any one of claims 1-4, wherein the anti-HER2 antibody-drug conjugate is administered to the subject at a dose of 1.5-9 mg/kg, 4.5-7.5 mg/kg, or 6-7.5 mg/kg each time; preferably, the anti-HER2 antibody-drug conjugate is administered to the subject at a dose of 6-7.5 mg/kg each time; or, the anti-HER2 antibody-drug conjugate is administered to the subject at a dose of 1.5 mg/kg, 3 mg/kg, 4.5 mg/kg, 6 mg/kg, 7.5 mg/kg, or 9 mg/kg each time.

6. The anti-HER2 antibody-drug conjugate for use in neoadjuvant therapy of breast cancer in a subject according to any one of claims 1-5, wherein one treatment cycle consists of 1 week, 2 weeks, 3 weeks, or 4 weeks, and the anti-HER2 antibody-drug conjugate is administered once per treatment cycle; preferably, one treatment cycle consists of 3 weeks, and the anti-HER2 antibody-drug conjugate is administered once per treatment cycle.

7. The anti-HER2 antibody-drug conjugate for use in neoadjuvant therapy of breast cancer in a subject according to any one of claims 1-6, wherein the anti-HER2 antibody-drug conjugate is administered for a total of 6-8 treatment cycles, preferably for a total of 6 treatment cycles or 8 treatment cycles.

8. The anti-HER2 antibody-drug conjugate for use in neoadjuvant therapy of breast cancer in a subject according to any one of claims 1-7, wherein the anti-HER2 antibody-drug conjugate is formulated into a formulation for intravenous injection or infusion.

9. The anti-HER2 antibody-drug conjugate for use in neoadjuvant therapy of breast cancer in a subject according to any one of claims 1-8, wherein the breast cancer is early-stage or locally advanced breast cancer; and/or
the breast cancer is invasive breast cancer, preferably unilateral invasive breast cancer.

10. The anti-HER2 antibody-drug conjugate for use in neoadjuvant therapy of breast cancer in a subject according to any one of claims 1-9, wherein the breast cancer is HER2-expressing breast cancer;
the breast cancer is HER2-positive breast cancer; and/or
the breast cancer is hormone receptor-positive or negative breast cancer.

11. The anti-HER2 antibody-drug conjugate for use in neoadjuvant therapy of breast cancer in a subject according to claim 10, wherein the HER2-positive breast cancer is breast cancer determined to have HER2 expression of 3+ by IHC or/and breast cancer determined to be positive for HER2 expression by ISH, or the HER2-positive breast cancer is breast cancer determined to have HER2 expression of 2+ by IHC and determined to be positive for HER2 expression by ISH;
preferably, the breast cancer is estrogen receptor- or/and progesterone receptor-positive breast cancer; or, the breast cancer is estrogen receptor- and progesterone receptor-negative breast cancer.

12. The anti-HER2 antibody-drug conjugate for use in neoadjuvant therapy of breast cancer in a subject according to any one of claims 1-11, wherein the breast cancer is HER2-positive early-stage or locally advanced breast cancer;
the breast cancer is HER2-positive and hormone receptor-positive early-stage or locally advanced breast cancer; or
the breast cancer is HER2-positive and hormone receptor-negative early-stage or locally advanced breast cancer.

13. The anti-HER2 antibody-drug conjugate for use in neoadjuvant therapy of breast cancer in a subject according to any one of claims 1-12, wherein the breast cancer has a clinical stage of T0 to T4, N1 to N3, and M0; or the breast cancer has a clinical stage of T2 to T4, N0, and M0.

14. The anti-HER2 antibody-drug conjugate for use in neoadjuvant therapy of breast cancer in a subject according to any one of claims 1-13, wherein the subject with breast cancer is eligible for surgical treatment after receiving neoadjuvant therapy;
optionally,
the subject with breast cancer has not previously received treatment for breast cancer; preferably, the subject with breast cancer has not previously received systemic therapy for breast cancer.

15. The anti-HER2 antibody-drug conjugate for use in neoadjuvant therapy of breast cancer in a subject according to any one of claims 1-14, wherein the anti-HER2 antibody-drug conjugate is formulated, together with one or more pharmaceutically acceptable excipients, into a pharmaceutical composition, and the pharmaceutical composition comprises a therapeutically effective amount of the anti-HER2 antibody-drug conjugate.
